# FASCICULE DE BREVET EUROPEEN

(11) **EP 2 561 335 B1**
(45) Date de publication et mention de la délivrance du brevet: **26.12.2018**
(21) Numéro de dépôt: 11731423.7
(22) Date de dépôt: 18.04.2011
(51) Int. Cl.: G01N 17/00, G01M 3/28

(54) **SUIVI DE LA VITESSE DE CORROSION D'UN CONDUIT METALLIQUE PARCOURU PAR UN FLUIDE CORROSIF**
VERFOLGUNG DER KORROSIONSRATE EINER VON EINER KORROSIVEN FLÜSSIGKEIT DURCHQUERTEN METALLLEITUNG
TRACKING OF THE RATE OF CORROSION OF A METAL CONDUIT TRAVERSED BY A CORROSIVE FLUID

(30) Priorité: 19.04.2010 FR 1052940
(43) Date de publication de la demande: 27.02.2013
(73) Titulaire: Total Raffinage France, 92400 Courbevoie (FR)
(72) Inventeur: ROUMEAU, Xavier, 77706 Beaumont TX (US); ALBINET, Benoît, 1640 Rhode-Saint-Genèse (BE); PELET, Christophe, F-76280 Angerville L'orcher (FR); HOULIER, Alain, F-76310 Sainte Adresse (FR); CASAJUS-GIL, Lionel, F-27210 Beuzeville (FR); FRACHISSE, Guilhem, F-76600 Le Havre (FR)
(74) Mandataire: Largeau, Béatrice
(86) Numéro de dépôt international: PCT/FR2011/050885
(87) Numéro de publication internationale: WO 2011/131897

(56) Documents cités:
- WO-A1-2008/067674
- FR-A1- 2 349 833
- GB-A- 2 312 279
- JP-A- 58 129 346
- JP-A- 61 025 047
- US-A- 5 279 169
- US-A- 5 392 661
- US-A- 6 058 765
- US-B1- 7 306 951

## Description

La présente invention se rapporte au suivi de la vitesse de corrosion d'un conduit métallique parcouru par un fluide corrosif, et peut en particulier trouver des applications dans l'industrie pétrolière.

Une paroi en un métal ferreux tel que l'acier au carbone (moins de 2% de carbone) peut se corroder en présence d'acides comme H₂S (hydrogène sulfuré) pour former du sulfure de fer ferreux (FeS) et de l'hydrogène, ce dernier pouvant être soit (i) directement libéré sous la forme de dihydrogène (H₂), soit (ii) rester adsorbé à la surface du métal à l'état monoatomique. L'hydrogène monoatomique peut circuler librement à travers les mailles du réseau cristallin et les joints de grains du métal, jusqu'à traverser entièrement la paroi. Dans ce cas, deux atomes d'hydrogène peuvent se combiner pour former du dihydrogène et quitter la paroi pour diffuser dans le milieu environnant, par exemple une atmosphère gazeuse, un liquide ou un autre solide.

D'autres acides peuvent provoquer le même type de corrosion, par exemple les acides naphténiques, qui sont des acides organiques comportant une ou plusieurs fonctions acide carboxylique, que nous désignerons par R-COOH. Les acides naphténiques réagissent avec le fer et produisent de l'hydrogène et des naphténates de fer, de formule (R-COO)Fe(OOC-R') dans lequel R et R' sont égaux ou différents ou bien R et R' sont liés ensemble pour former un seul substituant.

Les acides naphténiques se rencontrent dans certains pétroles bruts et sont à l'origine de problèmes de corrosion dans les installations de stockage et de raffinage pétrolier. Les acides naphténiques et l'hydrogène sulfuré sont présents indifféremment seuls ou en combinaison dans les produits pétroliers, qu'ils soient bruts, semi raffinés (bruts synthétiques) ou raffinés.

Les installations de stockage et de raffinage pétrolier comportent principalement des appareillages en acier, allié ou non, et sont soumis à de fortes contraintes de corrosion, en particulier lorsqu'il y a des zones présentant des gradients de température importants, par exemple dans des chaudières, des échangeurs thermiques, des colonnes de distillation, des réacteurs, des conduites ou des vannes. Des corrosions externes des installations par l'eau, l'oxygène de l'air ou d'autres espèces réactives telles que des polluants (acide sulfurique, H₂S, HCl, NH₃, etc.), et des corrosions internes par des fluides circulant dans les installations, tels que des pétroles bruts ou des produits raffinés, liquides ou gazeux, se produisent.

En fonctionnement, certaines parties des installations de stockage et de raffinage présentent des zones où la température de surface des matériaux peut dépasser 250°C et peut aller couramment au-delà de 450°C. Ces zones sont soumises à des contraintes de corrosion forte par les espèces acides précitées et nécessitent un suivi continu de la corrosion, qui peut, dans certains cas, être de plusieurs millimètres de métal corrodé par an. L'hydrogène issu de cette corrosion peut être capté par perméation à travers la paroi de l'installation. A cette fin, il est préférable de bien positionner le capteur à proximité de la zone de corrosion.

La détection et la mesure d'hydrogène issu de perméation sont connues en général et ont donné lieu à la commercialisation de dispositifs, dont certains sont basés sur :
- La collection d'hydrogène par aspiration et de la mesure de la pression d'hydrogène générée (WO 2008/067674, US 5,392,661, US 5,279,169) ;
- Le balayage d'une surface par un gaz ou de l'air, la collection dudit gaz ou air chargé en hydrogène et la mesure à l'aide d'un détecteur électrochimique ou par combustion (GB 2 312 279).

La détection et la mesure d'hydrogène issu de perméation est une méthode utile pour l'évaluation de la vitesse de corrosion des métaux, et en particulier des aciers, alliés ou non.

Les capteurs d'hydrogène commerciaux sont décrits comme ayant une plage de travail allant jusqu'à 500°C.

Il a été utilisé un système de mesure comprenant un capteur d'hydrogène connu, assemblé par pression sur une zone de mesure. Lors d'essais de dispositifs commerciaux, la demanderesse a observé que la mesure de la perméation d'hydrogène variait anormalement selon le type d'alliage métallique utilisé, en particulier lorsque la température de service était supérieure à 200-250°C.

Il existe donc un besoin pour un suivi de la vitesse de corrosion d'un conduit métallique parcouru par un fluide corrosif qui soit plus fiable, en particulier dans le cas de températures relativement élevées.

Il est proposé un procédé de suivi de la vitesse de corrosion d'un conduit métallique parcouru par un fluide corrosif. On prévoit un dispositif agencé de façon à former, lorsque ce dispositif est installé sur une paroi du conduit métallique, une chambre apte à recevoir de l'hydrogène gazeux issu par perméation à travers cette paroi de conduit. Le procédé comprend :
(i) une étape de traitement pour éliminer de la chambre une espèce métallique susceptible d'être réduite par l'hydrogène gazeux,
(ii) une étape de mesure d'une quantité d'hydrogène reçu dans la chambre, en vue d'une estimation de la vitesse de corrosion du conduit métallique.

En particulier, au cours de l'étape (i) de traitement, au moins la paroi de la chambre formée par ledit conduit est traitée sur toute sa surface, et l'étape de traitement est menée en balayant l'intérieur de la chambre avec un courant d'hydrogène gazeux.

Un fluide corrosif peut être un liquide ou un gaz, il peut être oxydant ou réducteur, acide ou basique. Par exemple, le fluide corrosif peut comprendre du H₂S et/ou un acide naphténique. Ainsi, un pétrole brut, selon sa composition, peut être un fluide corrosif au sens de l'invention.

L'espèce métallique susceptible d'être réduite par de l'hydrogène gazeux peut comprendre par exemple du Fe₂O₃ (hématite), ou autre.

Ce procédé permet de suivre la vitesse de corrosion de façon plus fiable, les valeurs de quantité d'hydrogène mesurées lors d'essais étant relativement proches des valeurs attendues.

L'étape de traitement peut par exemple être menée en décapant aussi la surface interne d'une ou plusieurs des parois de la chambre.

Le décapage peut être chimique, physico-chimique, mécanique, ou autre.

L'étape de traitement peut être effectuée avant l'installation du dispositif sur le conduit, en particulier en cas de décapage mécanique, effectué par exemple avec une brosse en métal. On peut par exemple prévoir de décaper une portion de paroi du conduit métallique, et d'appliquer sur la portion décapée un dispositif tel que décrit ci-dessus, choisi neuf par exemple.

Alternativement, l'étape de traitement peut être effectuée alors que le dispositif est déjà installé sur le conduit. On peut alors prévoir de traiter toutes les parois internes de la cavité en une seule opération.

Avantageusement, en fonctionnement, on balaye l'intérieur de la chambre avec un gaz vecteur inerte, et on mesure la quantité d'hydrogène contenue dans le gaz vecteur issu du balayage.

Dans la présente demande, on entend par « gaz inerte » un gaz exempt d'hydrogène gazeux et qui n'est pas susceptible à la température de fonctionnement de réagir avec le conduit métallique, ni avec les parois de la chambre, ni avec un appareil de mesure de la quantité d'hydrogène. Le gaz vecteur inerte peut par exemple comprendre de l'azote gazeux, de l'argon, de l'hélium et/ou le dioxyde de carbone. L'air (sec ou humide) n'est pas considéré comme un gaz inerte, dans le cas de conduits en métal.

On pourra tolérer des impuretés réactives, du type dioxygène ou eau gazeuse, dans le gaz vecteur inerte, dans une plage limitée, laquelle pourra être déterminée empiriquement. Par exemple, le gaz vecteur inerte comprend moins de 1% en masse d'impuretés réactives, et avantageusement moins de 0,1%.

De préférence, le gaz vecteur inerte sera toutefois exempt d'impuretés réactives, du type dioxygène ou eau gazeuse.

Alternativement, on fait le vide à l'intérieur de la chambre, et après un intervalle de temps donné, on mesure la pression à l'intérieur de la chambre afin d'estimer la quantité d'hydrogène gazeux présent.

Le gaz vecteur inerte peut également contenir de l'hydrogène. Dans ce cas, la proportion d'hydrogène dans le gaz vecteur inerte sera connue, soit par étalonnage, soit par mesure préalablement à son passage dans la chambre. L'utilisation d'un gaz vecteur comprenant de l'hydrogène permet de détecter une corrosion dans le système de détection et de mesure et/ou un défaut d'étanchéité. Un autre avantage de l'utilisation d'un gaz vecteur contenant de l'hydrogène est de protéger le système contre une corrosion.

Avantageusement, la concentration en hydrogène dans le gaz vecteur inerte est comprise entre 10 et 10000 ppm. La concentration en hydrogène dans le gaz vecteur sera adaptée en fonction du seuil bas de détection des moyens de mesure et de la concentration maximale en hydrogène admise au-delà de laquelle les moyens de mesure saturent.

Avantageusement, le procédé comprend en outre une étape de soudure du dispositif contre la paroi (externe) du conduit métallique de manière à étanchéifier la chambre. Il a été observé que cette étape conduisait à des résultats plus fiables encore. Sans vouloir être lié par une théorie, il n'est pas exclu qu'une étanchéité parfaite permette d'éviter des éventuelles fuites d'hydrogène hors de la chambre.

La soudure peut être effectuée avec un cordon de soudure, en particulier lorsque les parois du dispositif sont métalliques. Dans le cas d'un dispositif avec des parois en polymère, on pourra prévoir d'utiliser une colle compatible à la fois avec les polymères et les métaux.

Avantageusement, le procédé comprend en outre une étape consistant à corréler la quantité d'hydrogène mesurée à l'épaisseur du conduit corrodé. On peut par exemple utiliser à cet effet des résultats d'un étalonnage.

Avantageusement, le procédé comprend en outre une étape consistant à enregistrer la variation d'épaisseur du conduit en fonction du temps.

Avantageusement, on effectue un balayage avec un gaz inerte avant et/ou pendant et/ou après assemblage.

Il est en outre décrit un système de mesure de la vitesse de corrosion d'un conduit métallique comprenant :
- un dispositif agencé de façon à former, lorsque ce dispositif est installé sur une paroi du conduit métallique, une chambre apte à recevoir de l'hydrogène gazeux issu par perméation à travers ladite paroi dudit conduit métallique,
- des moyens de mesure d'une quantité d'hydrogène dans la chambre.

Les surfaces internes des parois de la chambre sont sensiblement exemptes espèces métalliques susceptibles d'être réduites par de l'hydrogène gazeux.

Ce système peut en particulier être obtenu après une étape d'élimination d'une ou plusieurs espèce(s) métallique(s) susceptible(s) d'être réduite(s) par de l'hydrogène gazeux.

Avantageusement, le dispositif est soudé sur la paroi du conduit métallique de manière à étanchéifier la chambre.

Avantageusement, le système comporte en outre :
- une arrivée et une sortie de gaz connectées sur la chambre pour balayer cette chambre avec un gaz vecteur inerte, et
- un analyseur de la quantité d'hydrogène présent dans le gaz collecté à la sortie de gaz.

Le gaz à la sortie de la chambre ayant entraîné l'hydrogène présent dans la chambre, cet hydrogène est collecté et la quantité collectée est mesurée.

L'analyseur de gaz peut par exemple comprendre un chromatographe en phase gazeuse, ou autre.

Avantageusement, le système comporte en outre un moyen de calcul (ou moyens de traitement) pour déduire des valeurs mesurées des valeurs d'épaisseur de paroi de conduit et éventuellement enregistrer la variation de cette épaisseur en fonction du temps.

Ce moyen de calcul peut par exemple comprendre un processeur, un CPU (de l'anglais « Central Processing Unit », un microcontrôleur, un ordinateur, ou autre.

On peut avantageusement prévoir d'intégrer en un seul appareil l'analyseur et le moyen de calcul, ainsi éventuellement que le dispositif formant une chambre.

De manière préférée, l'analyseur de gaz comprend un détecteur muni d'une membrane nickel-palladium. Il a été observé que des détecteurs comprenant une membrane nickel-palladium sont bien adaptés pour une utilisation dans une raffinerie de pétrole. Ces détecteurs sont bien adaptés pour une mesure dans une plage de température comprise entre -20°C et +90°C (bornes incluses). La présence de CO, CO₂ ou H₂S, pour des concentrations inférieures à 100ppm, n'interfèrent pas avec la mesure. En cas de pollution du détecteur par de l'oxygène ou par une mise à l'air accidentelle, la désorption de la membrane peut être réalisée au moyen d'un gaz inerte riche en hydrogène, par exemple 1% d'hydrogène dans de l'azote ou de l'argon.

Un détecteur à hydrogène à membrane nickel-palladium utilisable pour la présente application est commercialisé par la société H2Scan (Valencia, Etats-Unis d'Amérique). Dans le cas de l'utilisation d'un détecteur H2Scan, la concentration en hydrogène dans le gaz inerte sera adaptée pour être comprise entre 10ppm et 8000ppm (bornes incluses). Une concentration en hydrogène comprise entre 50ppm et 200ppm (bornes incluses) sera particulièrement préférée.

Les moyens de mesure du système (par exemple un analyseur) comprennent avantageusement un détecteur comprenant une membrane nickel-palladium.

Les moyens de traitement du système sont (i) connectés en permanence via une liaison filaire ou par radiofréquence aux moyens de mesure, ou (ii) ne sont pas directement connectés auxdits moyens de mesure et reçoivent les informations collectées par lesdits moyens de mesure via des moyens de déchargement de données, ces derniers étant conçus pour se connecter successivement (a) sur lesdits moyens de mesure pour l'acquisition des informations collectées par ces derniers, puis (b) sur lesdits moyens de traitement, pour la délivrance desdites informations collectées.

Il est en outre décrit une installation de stockage ou de raffinage de pétrole ou de produits pétroliers, comprenant un système tel que décrit ci-dessus.

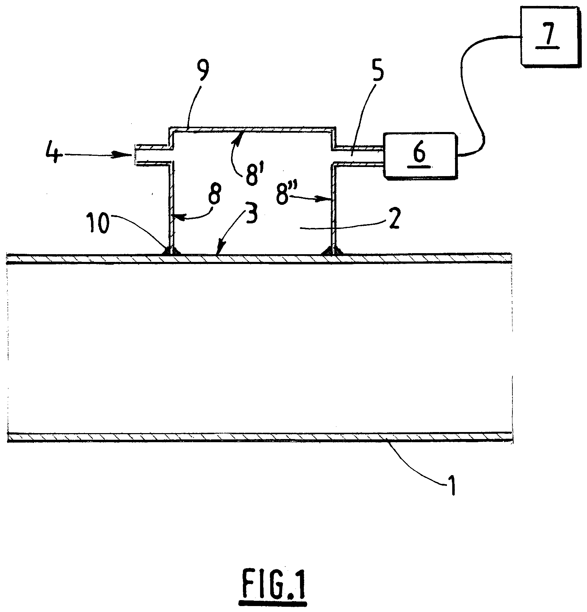

L'invention sera mieux comprise en référence à la figure 1, laquelle montre schématiquement un exemple de système selon un mode de réalisation.

Un conduit 1 est parcouru par un fluide corrosif, par exemple du pétrole.

Un dispositif 9 comporte des parois 8, 8', 8" définissant une cavité débouchante. Les parois 8, 8" ont une forme complémentaire de cette du conduit 1, de sorte que lorsque le dispositif 9 est soudé contre le conduit métallique 1, il est formé une chambre 2.

Dans la présente demande, on utilise parfois le terme « cavité » ou « cavité étanche » pour désigner cette chambre.

La cavité étanche 2 comporte une première paroi 3 qui est en fait une portion de la paroi du conduit métallique 1 dont on souhaite mesurer une quantité d'hydrogène issu de perméation. Cette paroi 3 est perméable à l'hydrogène, de sorte que la chambre 2 est susceptible de recevoir l'hydrogène issu de perméation.

Le dispositif 9 comporte une entrée 4 et une sortie 5 pour le balayage de la cavité 2 par un gaz vecteur inerte. Un courant de gaz vecteur peut ainsi être établi, de l'entrée 4 vers la sortie 5, ce courant étant ainsi susceptible d'entraîner vers la sortie 5 des éventuelles molécules de dihydrogène présentes dans la chambre.

Le dispositif 9 est agencé de façon à former un système fermé, de sorte que l'intérieur de la chambre soit isolé de l'air ambiant.

Des moyens de mesure, par exemple un chromatographe en phase gazeuse 6, permettent de mesurer une quantité de dihydrogène dans le gaz sortant de la cavité 2.

On peut prévoir de recueillir à des fins de mesure par le chromatographe 6 une partie seulement du gaz évacué de la chambre 2 via la sortie 5, le restant du gaz évacué de la chambre 2 via la sortie 5 étant alors entraîné par des moyens non représentés sur la figure 1, cette l'illustration étant très schématique.

Alternativement, on peut prévoir de recueillir l'ensemble du gaz évacué via la sortie 5.

Des moyens de traitement, par exemple un ordinateur 7, permettent de corréler quantité de dihydrogène mesurée par le chromatographe 6 et épaisseur de paroi, en tenant compte notamment des instants de mesure.

À cet effet, l'ordinateur 7 conserve en mémoire des données obtenues suites à un étalonnage d'un appareil de mesure de la quantité d'hydrogène produit en fonction du temps, tenant compte (i) de la surface de contact de la cavité étanche 2 avec le conduit 1, (ii) du débit de gaz vecteur, et (iii) de la température.

La cavité étanche 2 du dispositif de mesure est sensiblement exempte de corrosion sur ses faces internes 3, 8, 8', 8".

En effet, il a été observé que, dans le cas de conduits en métal, l'hydrogène issu de perméation mesuré était inférieur à la quantité théorique, et que l'enlèvement de la corrosion de surface du conduit qui se trouve en contact avec la chambre étanche permettait d'obtenir un résultat cohérent entre les quantités théorique et expérimentale d'hydrogène. Des conduits en métal du type acier au carbone sont particulièrement sensibles à ce type de traitement.

On peut noter qu'une fois le dispositif 9 installé sur le conduit 1, la cavité est isolée de l'air ambiant, de sorte qu'on peut s'attendre à ce que ses parois internes 3, 8, 8', 8" restent exemptes de corrosion.

Un cordon de soudure 10 permet d'étanchéifier la cavité 2, ce qui est particulièrement avantageux pour la détermination quantitative de l'hydrogène. En effet, l'étanchéité permet (i) d'éviter toute fuite d'hydrogène gaz et (ii) de préserver l'intérieur de la chambre 2 de toute oxydation accidentelle pouvant conduire à une diminution de la mesure d'hydrogène perméé.

Pour obtenir ce système, on peut prévoir que la zone 3 de paroi de conduit 1 faisant partie de la chambre étanche 2 subisse un traitement pour éliminer une corrosion éventuelle, avant l'installation du dispositif 9 sur le conduit 1. Par exemple, une partie de la surface externe du conduit 1 (incluant cette zone 3) est frottée à la paille de fer avant l'application du dispositif 9.

Une fois le dispositif 9 soudé sur le conduit 1, la chambre étanche 2 est balayée avec un courant d'hydrogène gazeux.

Le balayage avec un courant d'hydrogène gazeux peut être effectué à une température relativement élevée, par exemple supérieure à 250°C..

Puis la chambre étanche 2 est balayée avec un gaz inerte, c'est-à-dire exempt d'hydrogène et ne réagissant pas avec les différentes parties du système avec lesquelles ce gaz est amené à être en contact. Ce balayage permet en particulier d'évacuer des molécules d'hydrogène éventuellement présentes.

L'invention est utilisable dans tout type d'industrie soumise à des problèmes de corrosion, par exemple les industries pétrolière, chimique, biotechnologique et agro-alimentaire.

L'invention peut être avantageusement mise en oeuvre dans le cas de parois métalliques dont la température est supérieure à 200°C.

Dans la présente demande, on entend par « hydrogène gazeux » du dihydrogène et le terme « hydrogène » est parfois utilisé abusivement pour désigner du dihydrogène.

Un métal comprend la famille des métaux (corps purs) et des alliages de ceux-ci entre eux. Les métaux (corps purs) et alliages peuvent être combinés avec des non-métaux, par exemple C, Si, As, P, en toutes proportions.

## Revendications

1. Procédé de suivi de la vitesse de corrosion d'un conduit métallique parcouru par un fluide corrosif,
dans lequel on prévoit un dispositif agencé de façon à former, lorsque ledit dispositif est installé sur une paroi du conduit métallique, une chambre apte à recevoir de l'hydrogène gazeux issu par perméation à travers ladite paroi dudit conduit,
le procédé comprenant :
(i) une étape de traitement pour éliminer de la chambre une espèce métallique susceptible d'être réduite par l'hydrogène gazeux, au cours de laquelle au moins la paroi de la chambre formée par ledit conduit est traitée sur toute sa surface.
(ii) une étape de mesure d'une quantité d'hydrogène reçu dans la chambre, en vue d'une estimation de la vitesse de corrosion du conduit métallique,
**caractérisé en ce que** l'étape de traitement est menée en balayant l'intérieur de la chambre avec un courant d'hydrogène gazeux.

2. Procédé selon la revendication 1, comprenant en outre au cours de l'étape de mesure (ii) :
- une étape de balayage de l'intérieur de la chambre avec un gaz vecteur inerte,
- une étape de mesure de la quantité d'hydrogène contenue dans le gaz vecteur issu du balayage.

3. Procédé selon la revendication 2, dans lequel on balaye l'intérieur de la chambre avec le gaz vecteur inerte et de l'hydrogène.

4. Procédé selon la revendication 3, dans lequel la concentration en hydrogène dans le gaz vecteur inerte est comprise entre 10 et 10000 ppm.

5. Procédé selon l'une des revendications 1 à 4, dans lequel l'étape de mesure de ladite quantité d'hydrogène reçu dans la chambre est effectuée au moyen d'un détecteur comprenant une membrane nickel-palladium.

6. Procédé selon l'une des revendications 1 à 5, dans lequel l'étape de traitement est menée en décapant aussi des surfaces internes d'au moins une partie des parois de la chambre.

7. Procédé selon l'une des revendications 1 à 6, comprenant en outre une étape de soudure du dispositif contre la paroi du conduit métallique de manière à étanchéifier la cavité.

8. Procédé selon l'une des revendications 1 à 7, mis en oeuvre en utilisant un système de mesure de la vitesse de corrosion d'un conduit métallique (1) comprenant
- ledit dispositif (9),
- des moyens de mesure (6) d'une quantité d'hydrogène dans la chambre.

9. Procédé selon la revendication 8, dans lequel le système de mesure comporte en outre :
- une arrivée (4) et une sortie (5) de gaz connectées sur la chambre (2) pour le balayage de ladite chambre avec un gaz vecteur inerte, et
- un analyseur (6) de la quantité d'hydrogène présent dans le gaz collecté à la sortie de gaz.

10. Procédé selon la revendication 8 ou 9, dans lequel les moyens de mesure (6) comprennent un détecteur comprenant une membrane nickel-palladium.

11. Procédé selon l'une des revendications 8 à 10, dans lequel le système de mesure comporte des moyens de traitement (7) lesquels sont (i) connectés en permanence via une liaison filaire ou par radiofréquence aux moyens de mesure (6), ou (ii) ne sont pas directement connectés auxdits moyens de mesure (6) et reçoivent les informations collectées par lesdits moyens de mesure (6) via des moyens de déchargement de données (11), ces derniers se connectent successivement (a) sur lesdits moyens de mesure (6) pour l'acquisition des informations collectées par ces derniers, puis (b) sur lesdits moyens de traitement (7), pour la délivrance desdites informations collectées.

12. Procédé selon l'une quelconque des revendications 1 à 11 mis en oeuvre dans une installation de stockage ou de raffinage de pétrole ou de produits pétroliers.

## Patentansprüche

1. Verfahren zur Verfolgung der Korrosionsrate einer von einer korrosiven Flüssigkeit durchquerten Metallleitung,
wobei eine Vorrichtung bereitgestellt wird, welche eingerichtet ist, wenn die Vorrichtung an einer Wand der Metallleitung installiert wird, eine Kammer zu bilden, die geeignet ist, gasförmigen Wasserstoff aufzunehmen, der aus einer Permeation quer durch die Wand der Leitung stammt,
wobei das Verfahren umfasst:
(i) einen Behandlungsschritt, um aus der Kammer eine Metallart zu eliminieren, die durch gasförmigen Wasserstoff reduziert werden kann, während welchem mindestens eine Wand der von der Leitung gebildeten Kammer auf ihrer gesamten Fläche behandelt wird,
(ii) einen Messschritt einer Wasserstoffmenge, die in der Kammer aufgenommen wird, zur Schätzung der Korrosionsrate der Metallleitung,
**dadurch gekennzeichnet, dass** der Behandlungsschritt durch Abtasten des Inneren der Kammer mit einem gasförmigen Wasserstoffstrom durchgeführt wird.

2. Verfahren nach Anspruch 1,
außerdem umfassend während des Messschritts (ii):
- einen Schritt des Abtastens des Inneren der Kammer mit einem inerten Vektorgas,
- einen Schritt des Messens der Wasserstoffmenge, die in dem von der Abtastung stammenden Vektorgas enthalten ist.

3. Verfahren nach Anspruch 2,
wobei das Innere der Kammer mit dem inerten Vektorgas und Wasserstoff abgetastet wird.

4. Verfahren nach Anspruch 3,
wobei die Wasserstoffkonzentration in dem inerten Vektorgas zwischen 10 und 10000 ppm beträgt.

5. Verfahren nach einem der Ansprüche 1 bis 4,
wobei der Messschritt der Wasserstoffmenge, die in der Kammer aufgenommen wird, mit einem Detektor durchgeführt wird, der eine Nickel-Palladium-Membran umfasst.

6. Verfahren nach einem der Ansprüche 1 bis 5,
wobei der Behandlungsschritt durch Ätzen auch der Innenflächen mindestens eines Teils der Wände der Kammer durchgeführt wird.

7. Verfahren nach einem der Ansprüche 1 bis 6,
außerdem umfassend einen Schritt des Schweißens der Vorrichtung gegen die Wand der Metallleitung, um den Hohlraum abzudichten.

8. Verfahren nach einem der Ansprüche 1 bis 7,
welches unter Verwendung eines Messsystems der Korrosionsrate einer Metallleitung (1) durchgeführt wird, umfassend:
- die Vorrichtung (9),
- Messmittel (6) einer Wasserstoffmenge in der Kammer.

9. Verfahren nach Anspruch 8,
wobei das Messsystem außerdem umfasst:
- einen Gaseinlass (4) und einen Gasauslass (5), die mit der Kammer (2) zum Abtasten der Kammer mit einem inerten Vektorgas verbunden sind, und
- einen Analysator (6) der Wasserstoffmenge, die in dem am Gasauslass gesammelten Gas vorhanden ist.

10. Verfahren nach Anspruch 8 oder 9,
wobei die Messmittel (6) einen Detektor umfassen, der einen Nickel-Palladium-Membran umfasst.

11. Verfahren nach einem der Ansprüche 8 bis 10,
wobei das Messsystem Behandlungsmittel (7) umfasst, die (i) permanent über eine verdrahtete Verbindung oder durch eine Funkfrequenz mit den Messmitteln (6) verbunden sind, oder (ii) nicht direkt mit den Messmitteln (6) verbunden sind und Informationen, die von den Messmitteln (6) gesammelt werden, über Mittel (11) zum Liefern von Daten empfangen, wobei diese Letzteren aufeinanderfolgend (a) mit den Messmitteln (6) zum Erfassen von Informationen, die von diesen Letzteren gesammelt werden, dann (b) mit den Behandlungsmitteln (7) zum Liefern der gesammelten Informationen verbunden sind.

12. Verfahren nach einem der Ansprüche 1 bis 11,
welches in einer Lager- oder Raffinierungsanlage von Erdöl oder Erdölprodukten durchgeführt wird.

## Claims

1. A method of tracking the rate of corrosion of a metal pipe conveying a corrosive fluid,
in which a device is envisaged that is arranged so as to form, when said device is installed on a wall of the metal pipe, a chamber that is able to receive the gaseous hydrogen resulting from permeation through said wall of said pipe,
the method comprising:
(i) a treatment step for removing, from the chamber, a metallic species that could be reduced by the gaseous hydrogen, during which at least the chamber wall formed by said pipe is treated on its entire surface,
(ii) a step of measuring a quantity of hydrogen received in the chamber, with a view to estimating the rate of corrosion of the metal pipe,
**characterized in that** the treatment step is performed by scavenging the interior of the chamber with a stream of gaseous hydrogen.

2. The method as claimed in claim 1, further comprising during the treatment step:
- a step of scavenging the interior of the chamber with an inert carrier gas,
- a step of measuring the quantity of hydrogen contained in the carrier gas from scavenging.

3. The method as claimed in claim 2, in which the interior of the chamber is scavenged with the inert carrier gas and hydrogen.

4. The method as claimed in claim 3, in which the hydrogen concentration in the inert carrier gas is between 10 and 10000 ppm.

5. The method as claimed in one of claims 1 to 4, in which the step of measurement of said quantity of hydrogen received in the chamber is carried out by means of a detector comprising a nickel-palladium membrane.

6. The method as claimed in one of claims 1 to 5, in which the treatment step is performed by also scouring the internal surfaces of at least one portion of the walls of the chamber.

7. The method as claimed in one of claims 1 to 6, further comprising a step of welding the device onto the wall of the metal pipe so as to hermetically seal the cavity.

8. The method as claimed in one of claims 1 to 7, carried out using a system for measuring the rate of corrosion of a metal pipe (1) comprising
- said device (9),
- means (6) for measuring a quantity of hydrogen in the chamber.

9. The method as claimed in claim 8, in which the system for measuring further comprises:
- a gas inlet (4) and a gas outlet (5) connected to the chamber (2) for scavenging said chamber with an inert carrier gas, and
- an analyzer (6) of the quantity of hydrogen present in the gas collected at the gas outlet.

10. The method as claimed in one of claims 8 or 9, in which the measuring means (6) comprise a detector comprising a nickel-palladium membrane.

11. The method as claimed in one of claims 8 to 10, in which the system for measuring comprises processing means (7) which are (i) connected permanently via a wire link or by radio frequency to the measuring means (6), or (ii) are not directly connected to said measuring means (6) and receive the data collected by said measuring means (6) via data transfer means (11), the latter being designed to be connected successively (a) to said measuring means (6) for acquisition of the data collected by the latter, then (b) to said processing means (7), for retrieval of said collected data.

12. The method as claimed in any one of claims 1 to 11 carried out in an installation for storing or refining petroleum or petroleum products.
